# EUROPÄISCHE PATENTSCHRIFT

(11) **EP 0 895 463 B1**
(45) Veröffentlichungstag und Bekanntmachung des Hinweises auf die Patenterteilung: **27.06.2001**
(21) Anmeldenummer: 97914242.9
(22) Anmeldetag: 14.03.1997
(51) Int. Cl.: A61F 2/08

(54) **IMPLANTAT ZUR FESTLEGUNG EINER SEHNENERSATZPLASTIK**
IMPLANT TO SECURE A SINEW REPLACEMENT
IMPLANT PERMETTANT DE FIXER UN ELEMENT DE SUBSTITUTION DANS LE CADRE D'UNE TENOPLASTIE

(30) Priorität: 23.04.1996 DE 19616122
(43) Veröffentlichungstag der Anmeldung: 10.02.1999
(73) Patentinhaber: Aesculap AG & Co. KG, 78532 Tuttlingen (DE)
(72) Erfinder: EICHHORN, Jürgen, D-94360 Mitterfels (DE); GIORDANO, Nicola, D-78056 Villingen-Schwenningen (DE); KIENZLE, Karl-Ernst, D-78194 Immendingen (DE)
(74) Vertreter: Böhme, Ulrich, Dr. Dipl.-Phys.
(86) Internationale Anmeldenummer: EP9701292
(87) Internationale Veröffentlichungsnummer: WO9739700

(56) Entgegenhaltungen:
- EP-A- 0 317 408
- DE-U- 8 914 308
- DE-U- 9 002 844
- DE-U- 29 607 352
- US-A- 3 206 018
- US-A- 5 306 301

## Beschreibung

Die Erfindung betrifft ein Implantat zur Festlegung einer Sehnenersatzplastik an einem die Sehnenersatzplastik aufnehmenden Kanal im knienahen Bereich der Tibia gemäß den Merkmalen des Oberbegriffs des Anspruchs 1.

Es ist bekannt, zum Ersatz von zerstörten Kreuzbändern im Kniegelenk Sehnenersatzplastiken zu verwenden, die in den Femur und die Tibia im knienahen Bereich durchsetzende Kanäle eingesetzt werden und die dort mit an der Sehnenersatzplastik befestigten Fäden außerhalb des Femurs bzw. der Tibia festgelegt werden (US-A-5139520). Dabei werden zwar die Fäden am femurseitigen Austritt des entsprechenden Femurkanals mit Hilfe eines knopfähnlichen Implantates festgelegt, an der tibiaseitigen Austrittsöffnung des Tibia-Kanals jedoch ist es notwendig, unterhalb dieser Austrittsöffnung eine Knochenschraube in die Tibia einzusetzen und an dieser die Fäden festzulegen. Dadurch muß in der Tibia neben dem diese durchsetzenden Kanal eine weitere Öffnung vorgesehen werden, in die die Knochenschraube eingesetzt werden kann. Dies führt zu einer weiteren Schwächung der Tibia.

In der DE 90 02 844 U1 ist ein gattungsgemäßes Implantat beschrieben, welches aus einer Scheibe besteht, die im wesentlichen eben ausgebildet ist und in der Mitte einen nach beiden Seiten überstehenden Steg aufweist. Zu beiden Seiten des Steges sind Durchführungsöffnungen in der ebenen Scheibe angeordnet.

Ausgehend von diesem Stand der Technik ist es Aufgabe der Erfindung, ein Implantat der gattungsgemäßen Art so auszubilden, daß es zur Festlegung einer Sehnenersatzplastik geeignet ist, ohne daß das Implantat nach außen hin unnötig aufträgt.

Diese Aufgabe wird bei einem Implantat der eingangs beschriebenen Art erfindungsgemäß durch die Merkmale des Kennzeichnenden Teils des Anspruchs 1 gelöst.

Diese Ausgestaltung ist besonders günstig und ermöglicht einmal eine sichere Fixierung des Implantats in der Tibiabohrung, zum anderen trägt dieses Implantat nach außen hin äußerst wenig auf, so daß das Implantat nach der Operation ohne weiteres im Körper verbleiben kann. Dabei ist von Bedeutung, daß der ausgebauchte Bereich auf der einen Seite als Zentriervorsprung wirkt, der in die Knochenbohrung eintaucht, auf der anderen Seite dagegen die Verknotungen der Fäden aufnehmen kann, so daß auch diese nicht nach außen auftragen. Dadurch, daß dieser zentrale ausgebauchte Bereich von einem ebenen Randstreifen umgeben ist, stützt sich die Scheibe mit diesem Randstreifen am Knochen ab, und zwar im Bereich des die Bohrung im Knochen umgebenden Randes. Damit erhält man eine sichere zentrierte Festlegung einerseits und eine sehr enge Anlage des Implantats am Knochen andererseits.

Die Durchführungsöffnungen sind vorzugsweise im zentralen Bereich angeordnet, so daß die Fäden innerhalb der durch den zentralen Bereich gebildeten Vertiefung enden und dort verknotet werden können.

Die Außenumrandung des scheibenförmigen Implantats kann bei einer bevorzugten Ausführungsform kreisförmig ausgebildet sein, es ist aber auch günstig, wenn bei einer anderen Ausführungsform diese Außenumrandung oval ausgebildet ist.

Der ausgebauchte zentrale Bereich kann bei einer Ausführungsform im wesentlichen die Form eines Kugelabschnittes haben, in einer anderen bevorzugten Ausführungsform weist der ausgebauchte zentrale Bereich eine ovale Begrenzungslinie auf.

Günstig ist es, wenn das Implantat aus Titan besteht.

Ein Implantat mit einem zentralen ausgebauchten Bereich läßt sich besonders vorteilhaft als Ersatz einer bekannten tibiaseitigen Knochenschraube verwenden, grundsätzlich wäre es aber auch möglich, ein in dieser Weise ausgebildetes Implantat an anderen Knochenkanälen anzuordnen, um dort Sehnenersatzplastiken festzulegen, beispielsweise auch am Austritt des Femurkanals bei einer Kreuzbandersatzplastik.

Die nachfolgende Beschreibung bevorzugter Ausführungsformen der Erfindung dient im Zusammenhang mit der Zeichnung der näheren Erläuterung. Es zeigen:
- Figur 1:: eine Seitenansicht eines Kniegelenkes mit einer Kreuzbandersatzplastik in im Bereich der Knochenkanäle aufgebrochener Darstellung;
- Figur 2:: eine schematische Ansicht einer Sehnenersatzplastik mit an deren Enden festgelegten Fäden und mit Festlegungsimplantaten an beiden Enden;
- Figur 3:: eine Längsschnittansicht eines in die Knochenbohrung eingesetzten Implantats mit ovaler Formgebung und
- Figur 4:: eine Draufsicht auf das ovale Implantat der Figur 3.

Um in einem Kniegelenk 1 ein zerstörtes Kreuzband zu ersetzen, werden in den Femurkopf 2 und in den Tibiakopf 3 jeweils Längskanäle 4 bzw. 5 gebohrt, die bei gestrecktem Kniegelenk im wesentlichen miteinander ausgerichtet sind und die von der Vorderseite zur Rückseite des Kniegelenkes verlaufend dieses schräg durchsetzen.

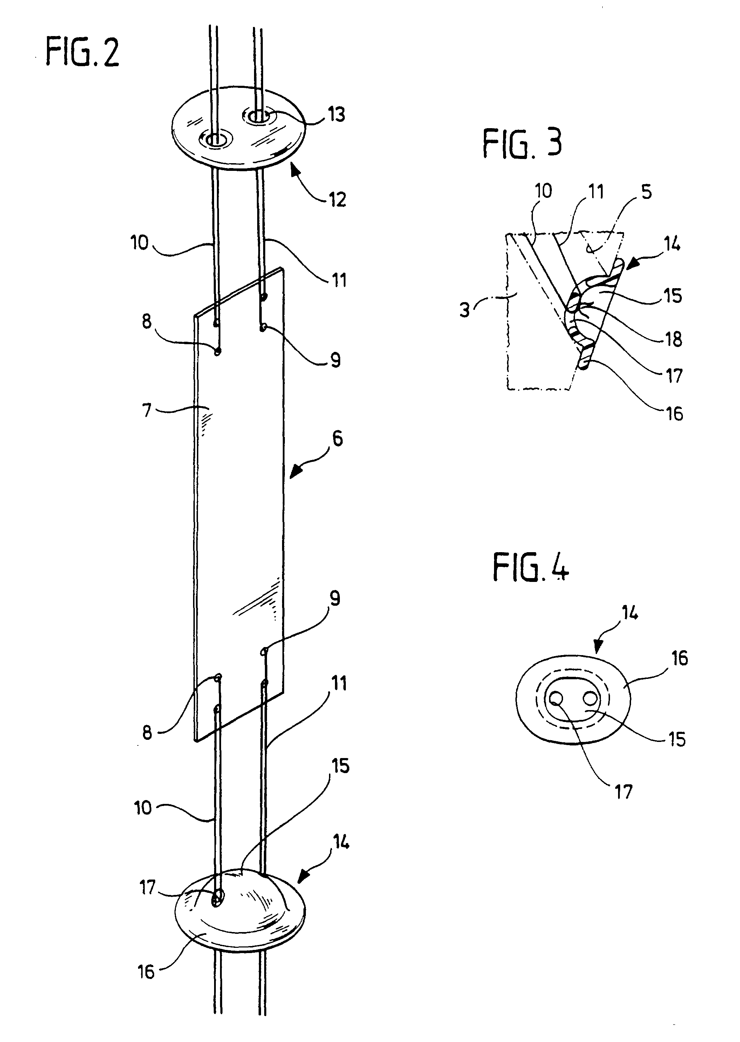

In die Längskanäle 4 und 5 wird eine Kreuzbandersatzplastik 6 eingezogen, die ein bandförmiges Stück 7 einer Sehnenersatzplastik umfaßt, beispielsweise ein Teil der Patellasehne, an deren Enden durch Öffnungen 8, 9 hindurchgezogen Fäden 10, 11 angeordnet sind, beispielsweise an beiden Enden zwei nebeneinander angeordnete Fäden 10 bzw. 11, wie dies aus der Darstellung der Figur 2 ersichtlich ist.

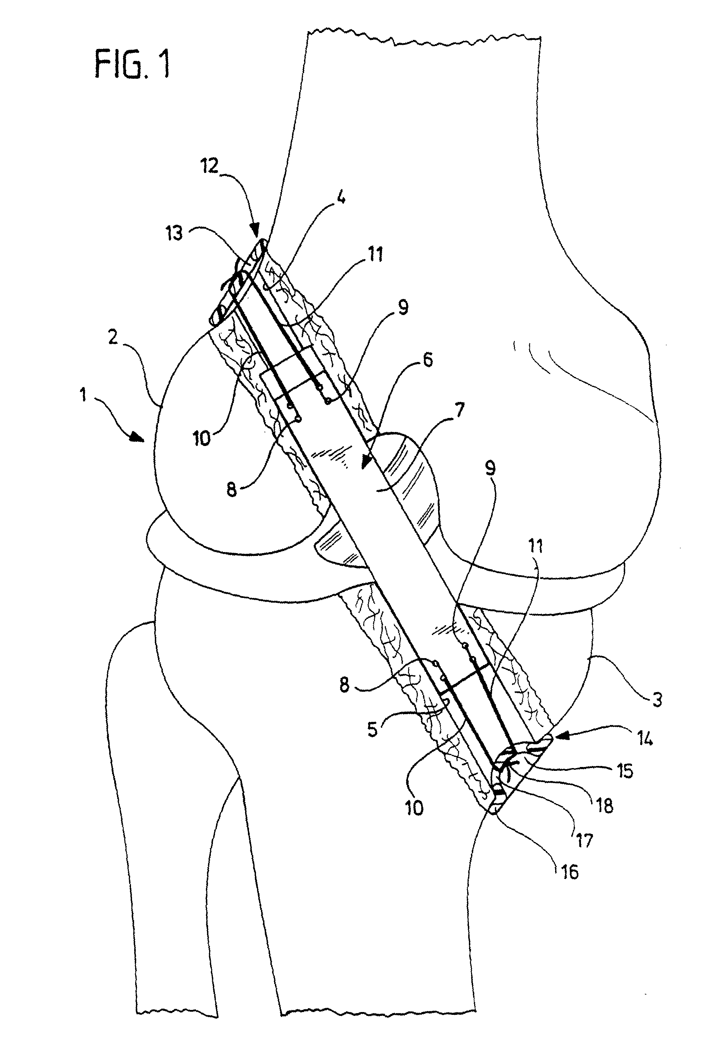

Zur Festlegung des Stückes 7 in den Längskanälen 4, 5 werden die Fäden 10, 11 aus den Längskanälen 4, 5 nach außen geführt und dort festgelegt. Am Austritt des Längskanals 4 ist ein knopfförmiges Implantat 12 mit zwei Durchstecköffnungen 13 angeordnet, durch die die Fäden 10, 11 hindurchgesteckt werden. Sie werden auf der knochenabgewandten Seite des Implantates 12 miteinander verknotet, so daß die Fäden 10, 11 an dem am Femurkopf 2 anliegenden Implantat 12 gehalten werden (Figur 1).

Auf der gegenüberliegenden Seite im Bereich des Austrittes des Längskanals 5 aus dem Tibiakopf 3 wird ein Implantat 14 verwendet, das einen kugelig ausgebauchten zentralen Bereich 15 und einen diesen ringförmig umgebenden, ebenen Randstreifen 16 umfaßt, so daß das Implantat 14 ein hutförmiges Aussehen erhält. Im zentralen Bereich 15 sind zwei Durchstecköffnungen 17 vorgesehen, durch die die Fäden 10, 11 hindurchgezogen werden.

Das Implantat 14 wird so in die Austrittsöffnung des Längskanals 5 geschoben, daß der nach innen ausgebauchte zentrale Bereich 15 in den Längskanal 5 eintaucht und dadurch das hutförmige Implantat 14 in der Austrittsöffnung zentriert. Dabei legt sich der Randstreifen 16 an die Außenfläche des Tibiakopfes 3 an, die die Austrittsöffnung des Längskanals 5 umgibt.

Die beiden die Durchstecköffnungen 17 durchsetzenden Fäden 10 und 11 werden auf der dem Längskanal 5 abgewandten Seite miteinander verknotet, und der dadurch entstehende Knoten 18 wird in der dem Knochen abgewandten Mulde des zentralen Bereiches 15 aufgenommen, so daß der Knoten 18 nicht nach außen über die Kontur des Implantates vorsteht (Figur 1).

Das Implantat 14 kann durch eine ausbauchende Verformung des zentralen Bereiches 15 hergestellt werden und kann vorzugsweise aus Titan bestehen und kann bei Bedarf im Körper verbleiben. Grundsätzlich wäre es auch möglich, das Implantat aus resorbierbarem Kunststoffmaterial herzustellen.

Bei dem Implantat der Figuren 1 und 2 ist die Umrandung kreisförmig ausgebildet, der zentrale Bereich ist kugelabschnittsförmig ausgebaucht, so daß dieses Implantat besonders geeignet ist, wenn die Austrittsöffnung des abzudeckenden Knochenkanals kreisförmig ist.

Bei dem Ausführungsbeispiel der Figuren 3 und 4, bei dem einander entsprechende Teile dieselben Bezugszeichen tragen, ist insofern eine Abänderung vorgenommen, als das Implantat oval ausgebildet ist, d.h. die Umrandung ist oval ausgebildet und ebenfalls der zentrale Bereich, der zur Ausbildung des Vorsprunges und der Vertiefung ausgebaucht ist. Diese Ausbauchung geht mit einer ovalen Umrandungslinie in den ebenen Randstreifen des schalenförmigen Implantates über, so daß der Vorsprung in einer Richtung länger ausgebildet ist als in der quer dazu verlaufenden Richtung. Dadurch ist dieses Implantat besonders zum Einsatz in Knochenkanäle geeignet, die mit einer ovalen Austrittsöffnung enden. In diesem Falle kann der Vorsprung formschlüssig in diese ovale Austrittsöffnung eingesetzt werden.

## Patentansprüche

1. Implantat (14) zur Festlegung einer Sehnenersatzplastik (7) an einem die Sehnenersatzplastik (7) aufnehmenden Kanal (5) im knienahen Bereich der Tibia (3) in Form einer Scheibe mit Durchführungsöffnungen (17) für mit der Sehnenersatzplastik (7) verbundene Fäden (10, 11), das so bemessen ist, daß es die Austrittsöffnung des Kanals (5) aus der Tibia (3) vollständig überdeckt, wobei das Implantat einen ausgebauchten zentralen Bereich (15) aufweist, der auf der dem Knochen (3) zugewandten Seite einen in den Kanal (5) eintauchbaren Vorsprung ausbildet, und wobei sich an den zentralen Bereich (15) außenseitig ein ebener, umlaufender Randstreifen (16) anschließt, dadurch gekennzeichnet, daß der zentrale Bereich (15) nur einseitig ausgebaucht ist und auf der dem Knochen (3) abgewandten Seite eine zentrale Vertiefung ausbildet.

2. Implantat nach Anspruch 1, dadurch gekennzeichnet, daß die Durchführungsöffnungen (17) im zentralen Bereich (15) angeordnet sind.

3. Implantat nach einem der voranstehenden Ansprüche, dadurch gekennzeichnet, daß die Scheibe eine kreisförmige Umrandung aufweist.

4. Implantat nach einem der Ansprüche 1 oder 2, dadurch gekennzeichnet, daß die Scheibe eine ovale Umrandung aufweist.

5. Implantat nach einem der voranstehenden Ansprüche, dadurch gekennzeichnet, daß der ausgebauchte zentrale Bereich (15) im wesentlichen die Form eines Kugelabschnittes hat.

6. Implantat nach einem der Ansprüche 1 bis 4, dadurch gekennzeichnet, daß der ausgebauchte zentrale Bereich (15) eine ovale Begrenzungslinie aufweist.

7. Implantat nach einem der voranstehenden Ansprüche, dadurch gekennzeichnet, daß es aus Titan besteht.

## Claims

1. An implant (14) for securing a tendon prosthesis (7) in a channel (5) accommodating the tendon prosthesis (7) in the region of the tibia (3), the implant (14) being in the form of a disc with through openings (17) for threads (10, 11) associated with the tendon prosthesis (7) and having dimensions such that it completely covers the exit opening of the channel (5) from the tibia (3), wherein the implant has a bulged central region (15) which, on the side facing the bone (3), forms a projection that can be embedded into the channel (5) and, in the central region on the outside, is joined to a flat, encircling marginal strip (16), characterised in that the central region (15) is bulged only on one side and forms a central depression on the side facing away from the bone (3).

2. An implant according to Claim 1, characterised in that the through openings (17) are arranged in the central region (15).

3. An implant according to one of the preceding claims, characterised in that the disc has a circular border.

4. An implant according to one of Claims 1 or 2, characterised in that the disc has an oval border.

5. An implant according to one of the preceding claims, characterised in that the bulged central region (15) has substantially the shape of a section of a sphere.

6. An implant according to one of Claims 1 to 4, characterised in that the bulged central region (15) has an oval delimiting line.

7. An implant according to one of the preceding claims, characterised in that it is made of titanium.

## Revendications

1. Implant (14) pour fixer un élément de substitution de ténoplastie (7) sur un canal (5) recevant l'élément de substitution de ténoplastie (7) dans la région du tibia (3) qui est proche du genou, sous forme d'un disque avec des ouvertures traversantes (17) pour des fils (10, 11) reliés à l'élément de substitution de ténoplastie (7), ledit implant ayant des dimensions telles qu'il recouvre totalement l'ouverture de sortie du canal (5) hors du tibia (3), l'implant présentant une région centrale (15) bombée qui forme sur le côté tourné vers l'os (3) une saillie susceptible de plonger dans le canal (5) et une bande de bordure (16) périphérique plane se raccordant sur le côté extérieur de la région centrale (15), caractérisé en ce que la région centrale (15) n'est bombée que d'un côté et forme un creux central sur le côté détourné de l'os (3).

2. Implant selon la revendication 1, caractérisé en ce que les ouvertures traversantes (17) sont agencées dans la région centrale (15).

3. Implant selon l'une des revendications précédentes, caractérisé en ce que le disque présente une bordure circulaire.

4. Implant selon l'une ou l'autre des revendications 1 et 2, caractérisé en ce que le disque présente une bordure ovale.

5. Implant selon l'une des revendications précédentes, caractérisé en ce que la région centrale (15) bombée a sensiblement la forme d'une calotte sphérique.

6. Implant selon l'une des revendications 1 à 4, caractérisé en ce que la région centrale (15) bombée présente une ligne de délimitation ovale.

7. Implant selon l'une des revendications précédentes, caractérisé en ce qu'il est constitué par du titane.
